# FASCICULE DE BREVET EUROPEEN

(11) **EP 0 893 133 B1**
(45) Date de publication et mention de la délivrance du brevet: **06.11.2002**
(21) Numéro de dépôt: 98410082.6
(22) Date de dépôt: 20.07.1998
(51) Int. Cl.: A61M 5/315, A61M 5/145

(54) **Dispositif d'injection d'un liquide à usage médical, seringue associée au dispositif et procédé de mise en place de la seringue**
Vorrichtung zur Injektion einer medizinischen Flüssigkeit, eine an diese Vorrichtung anschliessbare Spritze, sowie Verfahren zur Anwendung dieser Vorrichtung
Medical liquid injection device, syringe for use with the device and method of engaging the syringe

(30) Priorité: 24.07.1997 FR 9709661
(43) Date de publication de la demande: 27.01.1999
(73) Titulaire: Medex, 74941 Annecy le Vieux Cedex (FR)
(72) Inventeur: Lacroix, Jean-Pierre, 74940 Annecy le Vieux (FR)
(74) Mandataire: Gasquet, Denis

(56) Documents cités:
- EP-A- 0 346 950
- EP-A- 0 482 677
- EP-A- 0 584 531
- WO-A-95/26211
- US-A- 2 766 754
- US-A- 5 453 093

## Description

La présente invention concerne un dispositif d'injection d'un liquide à usage médical destiné au corps humain ou animal ; elle concerne également une seringue ainsi que le procédé de mise en place de la seringue sur le dispositif.

Les récents développements de la médecine ont conduit notamment à mettre au point différents procédés d'analyse et de contrôle de l'état des patients. Parmi ces procédés il existe les analyses faites après injection d'un produit de contraste. C'est par exemple le cas pour les analyses du type angiographie qui consistent à injecter un produit iodé dans les vaisseaux, veines et artères pour opacifier l'ensemble de ceux-ci ainsi que les tissus et organes imprégnés d'opacifiant et ainsi, par radio, déterminer les anomalies éventuelles, comme les thromboses, les embolies, les compressions et autres. Il peut s'agir aussi d'utilisation de produits de contraste pour réaliser un scanner ou en angiographie traditionnelle ou numérisée, l'injection veineuse ou artérielle devant se faire sous forme de bolus à vitesses allant généralement de 0,5 à 35 ml/s. Mais il existe aussi bien d'autres types d'injection sans pour autant que ce soient des injections de liquide de contraste. C'est par exemple le cas des transfusions ou des procédés de nutrition artificielle, notamment par le sang ou par voie digestive (entérale ou parentérale).

On connaît de nombreux dispositifs d'injection et notamment deux types d'injecteurs : les injecteurs à poche souple où le produit à injecter est conditionné directement dans des enveloppes souples, lesdites enveloppes étant destinées à être disposées dans une enceinte fermée de l'appareil que l'on met en pression ; et les injecteurs du type à seringue où le liquide à usage médical est conditionné dans une seringue destinée à être fixer à l'appareil pour être actionnée par celui-ci.

Les injecteurs à seringue tel que par exemple celui divulgué par 1 demande de brevet EP 0 584 531, présentent divers inconvénients liés à leurs éléments constitutifs ainsi qu'aux matériaux utilisés pour réaliser la seringue et son piston. La nécessité d'obtenir une étanchéité parfaite de l'enceinte interne de la seringue grâce à des joints en silicone disposés sur le piston provoque des effets de collage entre ledit piston et la paroi de la seringue pouvant occasionner un à-coup lors du démarrage de l'injection ainsi qu'une usure rapide du joint et donc de la seringue. De plus, la coopération entre le piston et l'appareil injecteur n'est pas satisfaisante en raison du jeu axial existant et la précision et les performances du dispositif d'injection sont moindres.

Ainsi, la présente invention a pour objectif de remédier aux inconvénients précités grâce à des moyens simples, fiables, de manipulation aisée et peu onéreux.

Ainsi, la présente invention concerne un dispositif d'injection selon la revendication 1.

De façon optionelle, le dispositif d'injection peut comporter les caractéristiques des revendications 2-5.

Par ailleurs, l'invention concerne également une seringue destinée à être utilisée dans un dispositif d'injection selon l'invention. Ladite seringue est définie dans la revendication 6 et peut comporter les caractéristiques définies dans les revendications 7-8.

Notons que l'invention concerne également un procédé de mise en place de la seringue sur l'appareil d'injection, ledit procédé comportant une étape principale qui consiste à effectuer un mouvement de rotation respectif entre le corps de seringue et le piston de la seringue.

D'autres caractéristiques et avantages de l'invention se dégageront de la description qui va suivre en regard des dessins annexés qui ne sont donnés qu'à titre d'exemples non limitatifs.

Les figures 1 à 7c illustrent un mode de réalisation préféré du dispositif d'injection selon l'invention et de certains de ses éléments constitutifs.

La figure 1 illustre en perspective le dispositif d'injection dans son ensemble.

La figure 2 représente en vue de dessus le dispositif d'injection dans son ensemble.

La figure 3a représente en vue latérale le corps de seringue du dispositif d'injection.

La figure 3b est une vue en bout selon F1 du corps de seringue de la figure 3a.

La figure 4a représente en vue latérale le fourreau porte seringue du dispositif d'injection.

La figure 4b est une vue en bout selon F2 du fourreau de la figure 4a.

La figure 5a représente en vue latérale l'organe de liaison et son élément de fixation.

La figure 5b représente l'organe de liaison dans une vue latérale selon F3 de la figure 5a.

La figure 5c représente une vue en bout selon F4 de l'organe de liaison.

La figure 6a illustre en vue latérale le piston de la seringue et son élément de fixation.

La figure 6b est une vue selon F5 de la figure 6a.

La figure 6c est une vue en bout selon F6 du piston.

Les figures 7a, 7b et 7c illustrent schématiquement en perspective les étapes du procédé de mise en place du fourreau et de la seringue sur l'appareil injecteur avant le début d'un cycle d'injection proprement dit.

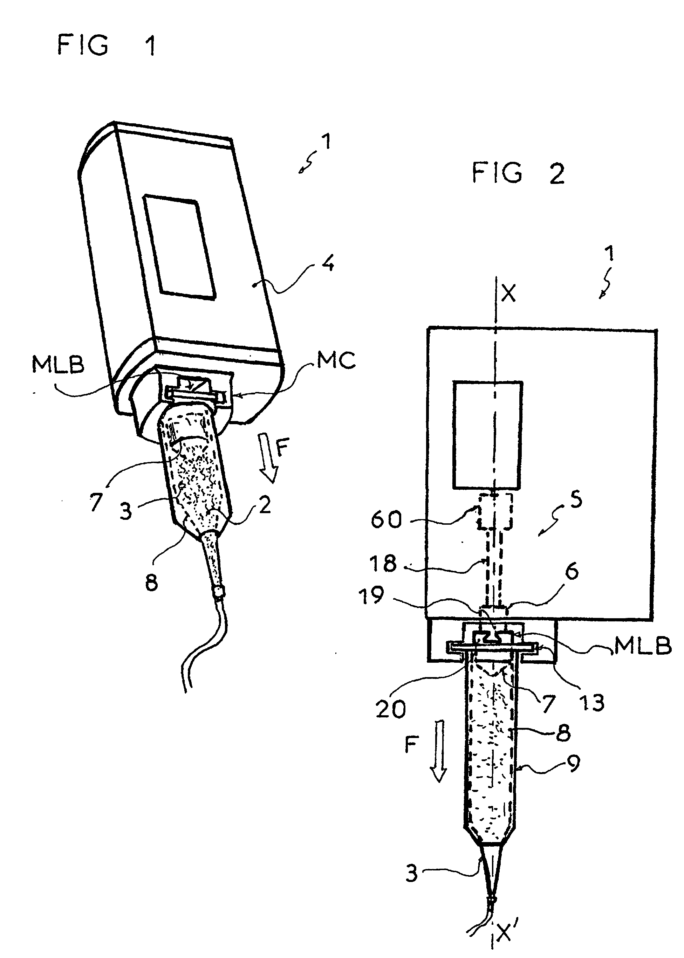

Selon l'invention, le dispositif d'injection illustré figure 1 portant la référence générale (1) est du type injecteur à seringue. Il comporte une seringue (3) à l'intérieur de laquelle se trouve le liquide médical (2) à injecter et un appareil d'injection (4) muni d'un dispositif d'actionnement (5). Le dispositif d'actionnement (5) comporte un moteur (60), un axe porteur (18) et un organe de liaison (6) destiné à coopérer avec le piston (7) de la seringue (3) pour transmettre un mouvement respectif de translation axiale (F) entre ledit piston et le corps de seringue (8) le long de l'axe longitudinal de révolution (XX') de la seringue, comme le montre la figure 2. Selon l'invention, il est prévu des moyens de liaison et de blocage (MLB) destinés à empêcher le pivotement autour de l'axe (XX') entre l'organe de liaison (6) et le piston (7), et à supprimer le jeu axial selon (XX') entre ces deux organes.

Selon le mode de réalisation préféré du dispositif d'injection (1) selon l'invention, celui-ci comporte un fourreau porte seringue (9) dans lequel est destinée à être disposée la seringue (3), ledit fourreau étant alors destiné à être solidarisé grâce à des moyens de connexion à l'appareil d'injection (4) de manière à permettre l'établissement de la liaison et de la coopération par les moyens de liaison et de blocage entre le piston (7) de la seringue (3) et l'organe de liaison (6) du dispositif d'actionnement (5) dudit appareil.

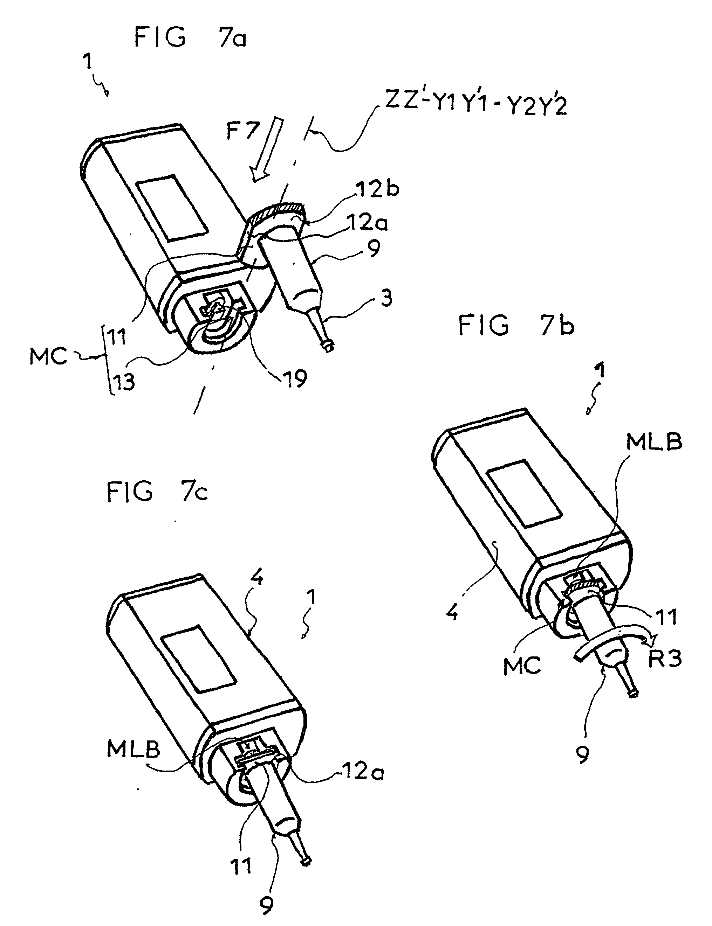

Selon l'invention, le procédé de mise en place de la seringue (3) sur l'appareil d'injection (4) est du type comportant une étape principale qui consiste à effectuer une rotation ou un pivotement respectif entre le corps de seringue (8) et le piston (7) muni de son joint. Cette rotation respective est avantageusement obtenue, comme le montrent les figures 7a à 7c, grâce aux moyens de liaison et de blocage (MLB) permettant de bloquer le piston (7) ainsi que, grâce aux moyens de connexion (MC) décrits ci-après et dont la mise en oeuvre s'effectue avec une rotation du fourreau (9) et donc du corps de seringue (8) qui pivote ainsi autour du piston (7).

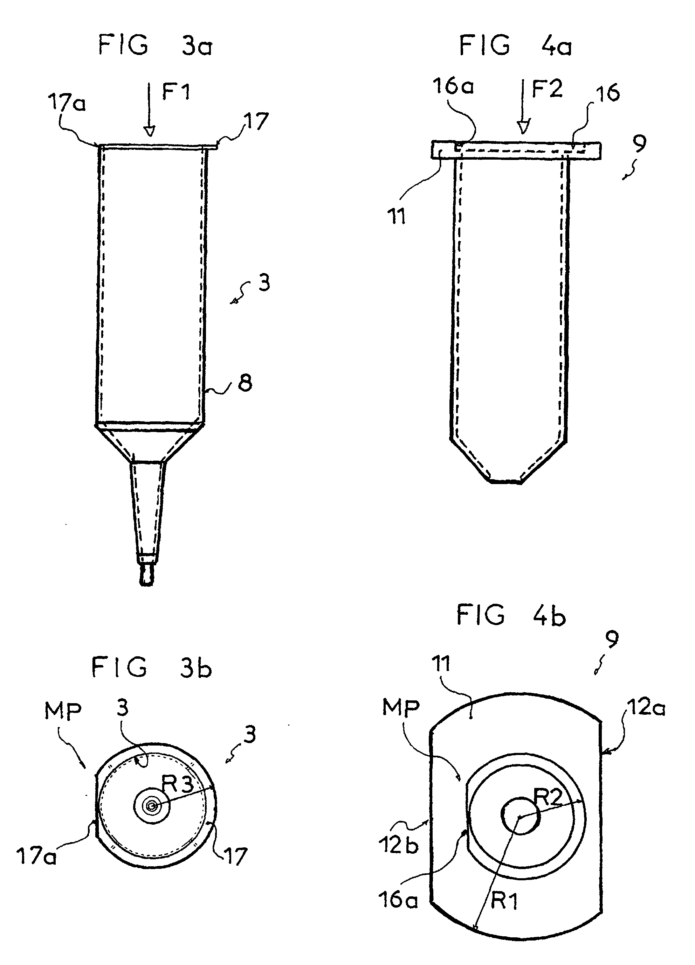

Le fourreau (9) et l'appareil d'injection (4) comportent ainsi chacun des moyens de connexion (MC) complémentaires destinés à permettre la mise en place du fourreau contenant la seringue (3) sur l'appareil (4). Lesdits moyens de connexion (MC) sont constitués en partie par un rebord supérieur (11) en forme de disque de rayon R1 disposé à l'extrémité supérieure du fourreau, ledit disque ayant été tronqué selon deux bords latéraux parallèles (12a, 12b), comme le montre la figure 4b. L'appareil d'injection (4) comporte à son extrémité inférieure, comme le montre la figure 7a, un logement de disque (13) constituant également un moyen de connexion (MC), ledit logement étant destiné à coopérer de manière connue en soi avec le rebord supérieur (11) du fourreau (9).

Notons que le logement (13) de l'appareil (4) comporte une ouverture d'introduction frontale destinée à permettre l'introduction du rebord (11) du fourreau (9) par coulissement (F7) selon un axe transversal (ZZ'), comme le montrent les figures 7a et 7b. Une fois ledit coulissement effectué, le fourreau (9) et le corps de seringue (8) effectuent de manière solidaire une rotation (R3) sensiblement d'un quart de tour autour de l'axe (XX') afin de mettre en place la connexion entre le fourreau (9) et l'appareil (4), en faisant coïncider de manière connue en soi les parties circulaires du rebord (11) et celles du logement (13) tel qu'illustré figure 7c. Des capteurs sont avantageusement prévus à l'intérieur du logement (13), ils sont destinés à vérifier la position du rebord (11) et donc du fourreau avant le lancement d'un cycle d'injection proprement dit, l'appareil ne pouvant être mis en fonctionnement que lorsque les capteurs constituant le dispositif de sécurité signalent une position adéquate du fourreau (9) et de sa seringue (3), appelée position de fonctionnement illustrée figure 1 et 7c.

Selon le mode de réalisation préféré du dispositif d'injection (1). Le fourreau (9) comporte avantageusement des moyens de positionnement (MP) destinés à permettre à la seringue (3) et plus particulièrement au corps de seringue (8) d'être disposée à l'intérieur du fourreau de manière à être bloquée en rotation autour de l'axe (XX') par rapport à celui-ci.

Le fourreau comporte à son extrémité supérieure sur la face supérieure de son rebord (11) un logement en creux (16) de forme cylindrique de rayon R2 égal au jeu près au rayon R3 du rebord supérieur (17) de la seringue (3) comme le montre les figures 3a, 3b, 4a et 4b. Les moyens de positionnement (MP) de la seringue dans le fourreau sont avantageusement constitués par deux plats (16a, 17a) identiques tronquant respectivement le logement en creux (16) du fourreau et le rebord supérieur (17) de la seringue (3) afin de solidariser le corps de seringue (8) et le fourreau (9) en rotation autour de l'axe de révolution (XX'). De ce fait, lors de l'étape principale du procédé de mise en place de la seringue (3) sur l'appareil d'injection (4), illustrée figures 7a, 7b et 7c, le corps de seringue (8) et le fourreau (9) subissent ensemble une rotation par rapport à l'appareil (4) autour de l'axe (XX') d'environ un quart de tour pour se retrouver en position de fonctionnement de manière à permettre la mise en fonctionnement du dispositif d'injection (1) et le lancement d'un cycle d'injection proprement dit tandis que le piston (7) et son joint restent fixe. Notons que lors de ce pivotement entre le corps de seringue et le piston, l'étanchéité est maintenu. Notons que le plat (16a) du logement (16) du fourreau (9) permettant d'indexer la position du corps de seringue est avantageusement parallèle aux bords latéraux (12a, 12b) du rebord supérieur (11) et se trouve ainsi positionné selon l'axe transversal (ZZ') du coulissement lors de la mise en place du fourreau porte seringue (9) sur l'appareil (4), cependant il va de soi que le plat pourrait former un angle par rapport aux bords latéraux. Il va également de soi que les moyens de connexion entre le fourreau et l'appareil pourraient être différents, de même les moyens de positionnement du corps de seringue dans le fourreau pourraient également être constitués par d'autres systèmes équivalents.

Selon le mode de réalisation préféré du dispositif d'injection (1), l'appareil injecteur (4) comporte un dispositif d'actionnement (5) destiné à provoquer le mouvement respectif de translation axiale entre le piston (7) et le corps de seringue (8). Ledit dispositif d'actionnement (5) est muni d'un organe de liaison (6) destiné à coopérer avec le piston (7) de la seringue (3) comme le montre la figure 2. Ladite coopération s'effectue par des moyens de liaison et de blocage (MLB) situés respectivement sur l'organe de liaison (6) et sur le piston (7) et destinés à solidariser ceux-ci de manière à empêcher tout pivotement autour de l'axe (XX') entre ledit organe et ledit piston. Notons que les moyens de liaison et de blocage (MLB) sont également avantageusement destinés à permettre d'empêcher le jeu axial selon l'axe (XX') entre l'organe de liaison et le piston.

Dans le mode de réalisation décrit à titre d'exemple, le fourreau (9) et le corps de seringue (8) sont disposés de manière connue en soi, de manière fixe sur l'appareil (4), le dispositif d'actionnement (5) provoque, lors du cycle d'injection, la translation de l'axe porteur (18) qui porte l'organe de liaison (6), ce mouvement de translation étant transmis au piston (7) qui se déplace ainsi dans le corps de seringue (8). Il va de soi que le mouvement respectif de translation axiale entre le corps de seringue et le piston pourrait être engendré non plus par un déplacement de l'organe de liaison, mais par le déplacement de la pièce sur laquelle est fixé le fourreau de manière à ce que le piston soit fixe et que le corps de seringue se déplace le long du piston.

Selon le mode de réalisation préféré du dispositif d'injection (1) selon l'invention, les moyens de liaison et de blocage (MLB) sont constitués par deux éléments de fixations complémentaires, un élément mâle (19) et un élément femelle (20). L'élément mâle (19) est formé par l'extrémité inférieure de l'organe de liaison (6) et est constitué par un volume en saillie présentant avantageusement au moins une surface de butée (21a) plane, tandis que l'élément femelle (20) est formé par l'extrémité supérieure du piston (7) et est constitué par un logement en creux dont la forme et les dimensions sont complémentaires de celles du volume en saillie de l'élément mâle, ledit logement comportant la (les) même(s) surface(s) de butée (21b) plane(s) que l'élément mâle (19). Lors de la mise en place du fourreau sur l'appareil, l'élément mâle (19) et l'élément femelle (20) sont destinés à se mettre en prise, les surfaces de butée respectives (21a, 21b) étant destinées à se positionner l'une contre l'autre de manière à solidariser en rotation l'élément mâle et l'élément femelle.

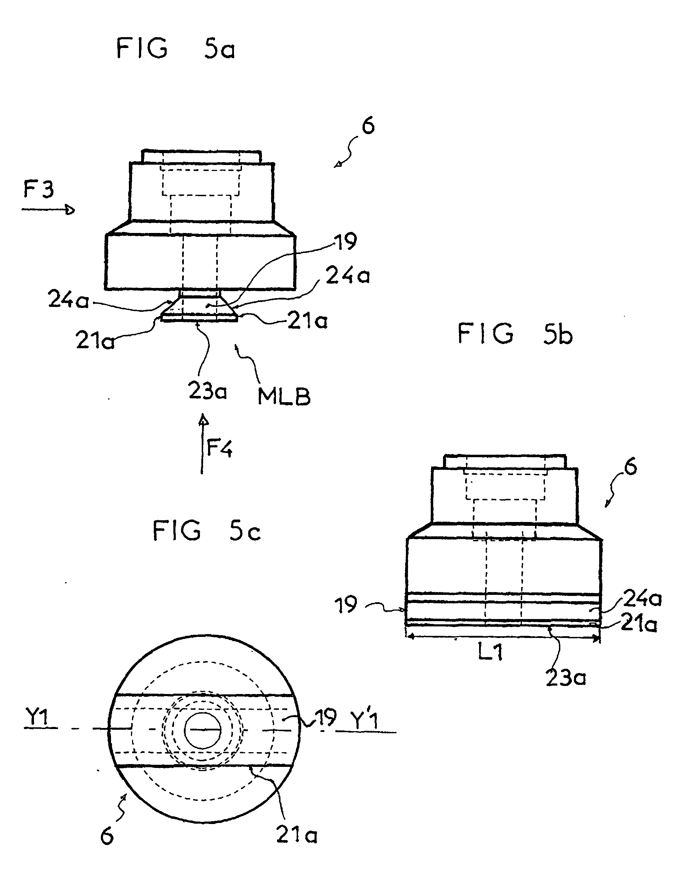

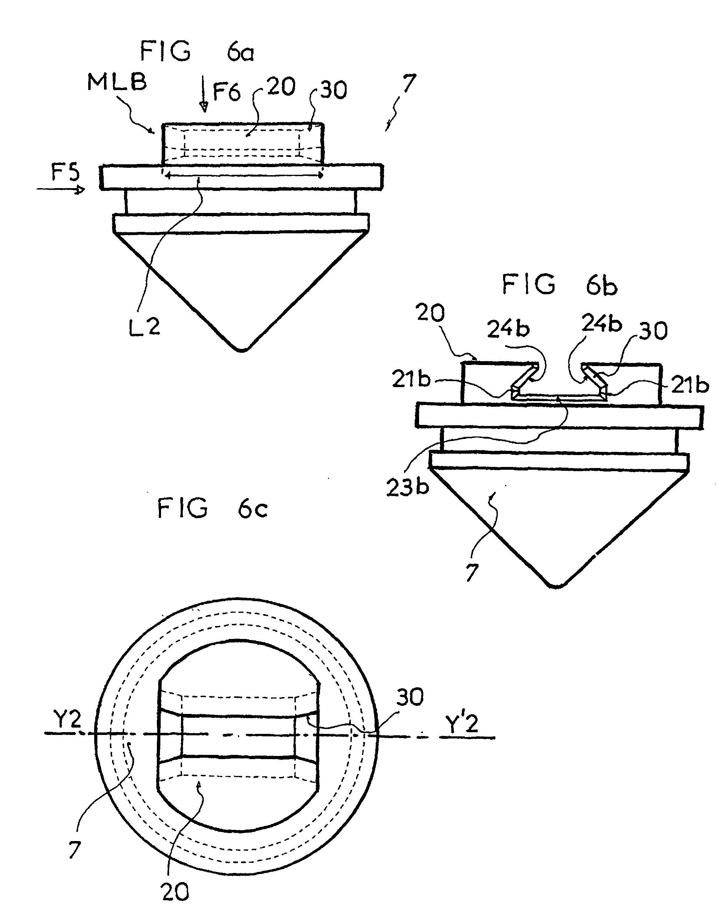

Selon le mode de réalisation préféré, et comme le montre les figures 5a, 5b, 5c, 6a, 6b et 6c, le volume en saillie et le logement en creux complémentaire possèdent avantageusement un profil transversal trapézoïdal de même dimension s'étendant respectivement selon deux axes longitudinaux (Y1Y'1) et (Y2Y'2), et ont ainsi la forme d'une queue d'aronde, les surfaces de butée (21a, 21b), dont la coopération est destinée à empêcher tout pivotement, étant constituées par les parois latérales des éléments mâle et femelle (19, 20) en forme de queue d'aronde. Les parois inférieures (23a, 23b) et les parois supérieures inclinées (24a, 24b) des éléments mâle et femelle (19, 20) coopèrent ensemble afin d'éliminer le jeu axial entre l'organe de liaison (6) et le piston (7) et constituent ainsi des surfaces d'appui de transmission complémentaires destinées notamment à transmettre le mouvement de l'organe de liaison au piston. Il va de soi que l'élément mâle pourrait être disposé sur le piston et l'élément femelle sur l'organe de liaison.

Selon le mode de réalisation préféré de l'invention, le profil transversal trapézoïdal en forme de queue d'aronde des éléments mâle et femelle (19, 20) est identique. Les longueurs respectives (L1, L2) sur lesquelles s'étendent lesdits profils sont inférieures au diamètre interne du corps de seringue (8), et notamment pour l'élément situé sur l'organe de liaison (6), à savoir, l'élément mâle (19), de manière à ce que l'organe de liaison puisse s'introduire dans ledit corps de seringue (8) lors de son mouvement de translation. Par ailleurs, les longueurs précitées (L1, L2) sur lesquelles s'étend le profil trapézoïdal peuvent être avantageusement identiques.

Notons que, de manière connue en soi, les éléments mâle et femelle (19, 20) portés par le piston (7) et l'organe de liaison (6) ne peuvent être mis en position de coopération que par coulissement selon les axes longitudinaux (Y1Y'1) et (Y2Y'2) de leurs profils, cette mise en position s'effectuant de manière simultanée avantageusement lors de la mise en place par coulissement du fourreau (9) sur l'appareil injecteur (4) selon l'axe transversal (ZZ') comme le montre la figure 7a.

Selon le mode de réalisation préféré de l'invention, l'organe de liaison (6) est disposé de manière fixe sur l'axe porteur (18) du dispositif d'actionnement (5) de manière à ce que l'axe longitudinal (Y1Y'1) le long duquel s'étend le profil trapézoïdal du volume en forme de queue d'aronde soit positionné dans la direction du coulissement (F2) destiné à la mise en place du rebord supérieur (11) du fourreau (9) dans le logement (13) de l'appareil (4), c'est-à-dire selon l'axe transversal (ZZ').

Ainsi, selon l'invention, la seringue (3) comporte un piston (7) dont l'axe longitudinal (Y2Y'2) du logement femelle (20) décrit précédemment est disposé parallèlement au plat (17a) du rebord supérieur (17) du corps de seringue (8). De ce fait, lors de la mise en place du fourreau (9), les éléments mâle et femelle (19, 20) sont disposés selon l'axe de coulissement et peuvent ainsi être mis en prise de manière connue en soi afin de pouvoir coopérer. Notons que la forme des éléments mâle et femelle permet de supprimer le jeu axial présent dans les injecteurs classiques grâce à leurs surfaces d'appui de transmission complémentaires (23a, 23b, 24a, 24b). De plus, lors du pivotement du fourreau (9) destiné à le positionner en position de fonctionnement, le corps de seringue (8) est entraîné dans la rotation (R3) d'un quart de tour grâce à la coopération des moyens de positionnement (MP) du fourreau et du corps de seringue. Ainsi, le piston bloqué en rotation par les moyens de liaison et de blocage (MLB) reste fixe, tandis que le corps de seringue (8) pivote autour de lui de manière à permettre le décollage du joint silicone du piston (7) de la paroi du corps de seringue. Le cycle d'injection peut ensuite démarrer sans être gêné ni par le jeu axial, ni par l'effet de collage du joint contre la paroi. Le piston (7) et son joint sont avantageusement réalisés de manière à ce que l'étanchéité soit maintenue pendant le pivotement du corps de seringue autour du piston. Il va de soi que l'amplitude du pivotement respectif entre le piston et le corps de seringue pourrait être différente, comme, par exemple, d'un demi tour.

Selon le mode de réalisation préféré de l'invention, le corps de seringue (8) est avantageusement réalisé en polypropylène, tandis que le piston (7) et le fourreau (9) sont réalisés en polycarbonate, le piston étant revêtu d'un joint en silicone dans sa partie inférieure.

Notons également que le logement de profil trapézoïdal de l'élément femelle (20) disposé sur le piston (7) comporte à ses extrémités des chanfreins d'entrée (30) destinés à autoriser un léger écart dans le positionnement de l'axe (Y2Y'2) dudit profil par rapport au plat (17a) du rebord supérieur (17) du corps de seringue lors du conditionnement du produit dans la seringue. Les chanfreins d'entrée (30) permettent avantageusement, lors du coulissement (F2) destiné à mettre en place le fourreau et la seringue sur l'appareil, de corriger l'écart angulaire du piston (7), l'élément mâle (19) forçant l'élément femelle (20) à pivoter légèrement afin de repositionner son logement selon l'axe longitudinal de coulissement (ZZ').

## Revendications

1. Dispositif d'injection (1) d'un liquide à usage médical (2) sur lequel une seringue (3) est destinée à être montée, ledit dispositif d'injection comportant un dispositif d'actionnement (5) muni d'un organe de liaison (6) destiné à coopérer avec le piston (7) de la seringue (3) de manière à ce que le dispositif d'actionnement (5) puisse transmettre un mouvement respectif de translation axiale selon l'axe longitudinal (XX') entre le piston (7) et le corps de seringue (8) de la seringue (3), ledit dispositif comportant des moyens de liaison et de blocage (MLB) destinés à empêcher tout pivotement autour de l'axe (XX') entre l'organe de liaison (6) et le piston (7), lesdits moyens étant constitués par un élément de fixation (19) disposé à l'extrémité de l'organe de liaison (6) et destiné à coopérer avec un élément complémentaire, disposé à l'extrémité supérieure du piston (7), ledit élément de fixation comportant un élément mâle (19) présentant un volume en saillie destiné à coopérer avec un élément femelle (20) muni d'un logement en creux, de forme identique et complémentaire à celle du volume en saillie, **caractérisé en ce que** le volume en saillie (19) possède un profil transversal trapézoïdal s'étendant le long d'un axe longitudinal (Y1Y'1) et a ainsi la forme d'une queue d'aronde.

2. Dispositif d'injection (1) d'un liquide à usage médical (2) selon la revendication 1, **caractérisé en ce que** l'élément de fixation (19) présente au moins une surface de butée (21a, 21b) plane destinée à coopérer avec une respective surface pleine d'un élément de fixation de la seringue de manière à empêcher le pivotement entre le piston (7) et l'organe de liaison (6), la (les) surface(s) de butée (21a, 21b) étant constituée(s) par les parois latérales du volume.

3. Dispositif d'injection (1) d'un liquide à usage médical (2) selon la revendication 1 ou la revendication 2, **caractérisé en ce qu'**il comporte un fourreau porte seringue (9) destiné à être positionné sur l'appareil par coulissement selon un axe longitudinal de coulissement (ZZ') orthogonal à l'axe (XX') de révolution de la seringue, des moyens de positionnement (MP) de la seringue (3) dans le fourreau (9) étant prévus de manière à ce que l'axe longitudinal (Y1Y'1) ou (Y2Y'2) de l'élément de fixation mâle ou femelle du piston (7) de la seringue soit confondu avec l'axe de coulissement (ZZ').

4. Dispositif d'injection (1) d'un liquide à usage médical (2) selon la revendication 3, **caractérisé en ce que** le fourreau (9) comporte à son extrémité supérieure sur la face supérieure de son rebord (11) un logement en creux (16) de forme cylindrique de rayon égal au jeu près au rayon du rebord supérieur (17) de la seringue (3).

5. Dispositif d'injection (1) d'un liquide à usage médical (2) selon la revendication 4, **caractérisé en ce que** les moyens de positionnement (MP) de la seringue dans le fourreau sont constitués par un plat (16a, 17a) tronquant le logement en creux (16) du fourreau et (9) afin de solidariser le corps de seringue (8) et le fourreau (9) en rotation autour de l'axe de révolution (XX'), le rebord supérieur (17) de la seringue étant aussi tronqué par un plan, de façon identique au fourreau.

6. Seringue (3) destinée à être utilisée dans un dispositif d'injection (1) selon l'une quelconque des revendications précédentes **caractérisée en ce qu'**elle comporte sur la face supérieure du piston (7) un élément de fixation femelle (20) formé par un logement en creux de profil transversal trapézoïdal s'étendant le long d'un axe longitudinal (Y2Y'2), ayant ainsi la forme d'une queue d'aronde.

7. Seringue (3) selon la revendication 6, **caractérisée en ce que** le corps de seringue (8) comporte un rebord supérieur (17) tronqué latéralement par un plat (17a) disposé parallèlement à l'axe longitudinal (Y2Y'2) de l'élément de fixation femelle (20) du piston (7).

8. Seringue (3) selon la revendication 6 ou 7, **caractérisée en ce que** le piston (7) est réalisé en polycarbonate et **en ce que** le corps de seringue (8) est réalisé en polypropylène.

9. Procédé de mise en place d'une seringue (3) selon l'une quelconque des revendications 6 à 9 sur l'appareil d'injection (4) d'un dispositif d'injection (1) selon l'une quelconque des revendications 1 à 5, **caractérisé en ce qu'**il comporte une étape principale qui consiste à effectuer un mouvement de rotation respectif entre le corps de seringue (8) et le piston (7) de la seringue (3).

## Patentansprüche

1. Einspritzvorrichtung (1) zur Injektion einer medizinischen Flüssigkeit (2), auf die eine Spritze (3) montierbar ist, wobei die Einspritzvorrichtung eine Betätigungsvorrichtung (5) umfasst, die ein Verbindungselement (6) aufweist, das mit dem Kolben (7) der Spritze (3) zusammenwirken soll, so dass die Betätigungsvorrichtung (5) zwischen dem Kolben (7) und dem Spritzenkörper (8) der Spritze (3) eine jeweilige Verschiebung in Axialrichtung entlang der Längsachse (XX') übertragen kann, wobei die Vorrichtung Verbindungs- und Blockierungsmittel (MLB) aufweist, mit denen ein Schwenken um die Achse (XX') zwischen dem Verbindungselement (6) und dem Kolben (7) verhindert wird, wobei die Mittel aus einem am Ende des Verbindungselements (6) angeordneten Befestigungselement (19) bestehen, das mit einem ergänzenden, am oberen Ende des Kolbens (7) angeordneten Element zusammenwirken soll, wobei das Befestigungselement ein männliches Teil (19) mit einem vorspringenden Raum umfasst, das mit einem weiblichen Teil (20) mit einer vertieften Aufnahme, die in ihrer Form mit der des vorspringenden Raums identisch ist und diese ergänzt, zusammenwirken soll, **dadurch gekennzeichnet, dass** der vorspringende Raum (19) ein trapezförmiges Querprofil aufweist, das sich entlang einer Längsachse (Y1Y'1) erstreckt, und dadurch die Form eines Schwalbenschwanzes aufweist.

2. Einspritzvorrichtung (1) zur Injektion einer medizinischen Flüssigkeit (2) nach Anspruch 1, **dadurch gekennzeichnet, dass** das Befestigungselement (19) mindestens eine ebene Anschlagfläche (21a, 21b) aufweist, die mit einer entsprechenden ebenen Fläche eines Befestigungselements der Spritze derart zusammenwirken soll, dass eine Schwenkbewegung zwischen dem Kolben (7) und dem Verbindungselement (6) verhindert wird, wobei die Anschlagfläche(n) (21a, 21b) durch die Seitenwände des Raums gebildet wird (werden).

3. Einspritzvorrichtung (1) zur Injektion einer medizinischen Flüssigkeit (2) nach Anspruch 1 oder 2, **dadurch gekennzeichnet, dass** sie einen hülsenförmigen Spritzenhalter (9) aufweist, der entlang einer senkrecht zur Drehachse (XX') der Spritze längsverlaufenden Verschiebungsachse (ZZ') verschieblich auf dem Gerät positioniert werden soll, wobei Positioniermittel (MP) zum Positionieren der Spritze (3) in dem Spritzenhalter (9) vorgesehen sind, so dass die Längsachse (Y1Y'1) oder (Y2Y'2) des männlichen oder weiblichen Befestigungselements des Spritzenkolbens (7) sich mit der Verschiebungsachse (ZZ') deckt.

4. Einspritzvorrichtung (1) zur Injektion einer medizinischen Flüssigkeit (2) nach Anspruch 3, **dadurch gekennzeichnet, dass** der Spritzenhalter (9) an seinem oberen Ende auf der oberen Seite seiner Kante (11) eine vertiefte, zylindrische Aufnahme (16) aufweist, deren Radius bis auf das Spiel dem Radius der Oberkante (17) der Spritze (3) entspricht.

5. Einspritzvorrichtung (1) zur Injektion einer medizinischen Flüssigkeit (2) nach Anspruch 4, **dadurch gekennzeichnet, dass** die Positioniermittel (MP) zum Positionieren der Spritze im Spritzenhalter mit einer Flachstelle (16a, 17a) ausgebildet sind, welche die vertiefte Aufnahme (16) des Spritzenhalters (9) abflacht, um den Spritzenkörper (8) und den Spritzenhalter (9), die um die Drehachse (XX') drehen, fest miteinander zu verbinden, wobei die Oberkante (17) der Spritze in derselben Weise wie der Spritzenhalter durch eine Flachstelle abgeflacht wird.

6. Spritze (3) zur Verwendung in einer Einspritzvorrichtung (1) nach einem der vorstehenden Ansprüche, **dadurch gekennzeichnet, dass** sie auf der Oberseite des Kolbens (7) ein weibliches Befestigungselement (20) aufweist, das durch eine entlang einer Längsachse (Y2Y'2) sich erstreckende, vertiefte Aufnahme mit trapezförmigem Querprofil gebildet wird, die somit die Form eines Schwalbenschwanzes aufweist.

7. Spritze (3) nach Anspruch 6, **dadurch gekennzeichnet, dass** der Spritzenkörper (8) eine seitlich durch eine Flachstelle (17a) abgeflachte Oberkante (17) aufweist, die parallel zur Längsachse (Y2Y'2) des weiblichen Befestigungselements (20) des Kolbens (7) angeordnet ist.

8. Spritze (3) nach Anspruch 6 oder 7, **dadurch gekennzeichnet, dass** der Kolben (7) aus Polykarbonat und der Spritzenkörper (8) aus Polypropylen hergestellt sind.

9. Verfahren zum Einsetzen einer Spritze (3) nach einem der Ansprüche 6 bis 9 in ein Einspritzgerät (4) einer Einspritzvorrichtung (1) nach einem der Ansprüche 1 bis 5, **dadurch gekennzeichnet, dass** es einen Hauptschritt umfasst, der darin besteht, eine Drehbewegung zwischen dem Spritzenkörper (8) und dem Kolben (7) der Spritze (3) auszuführen.

## Claims

1. Device (1) for injecting a liquid for medical use (2), on which a syringe (3) is adapted to be mounted, said injecting device comprising an actuating device (5) provided with a connecting member (6) intended to cooperate with the plunger (7) of the syringe (3) so that the actuating device (5) can transmit a respect movement of axial translation along the longitudinal axis (XX') between the plunger (7) and the body (8) of the syringe (3), said device comprising connecting and blocking means (MLB) intended to prevent any pivoting about the axis (XX') between the connecting member (6) and the plunger (7), said means consisting of a fixing element (19) arranged at the end of the connecting. member (6) and adapted to cooperate with a complementary element arranged at the upper end of the plunger (7), said fixing element comprising a male element (19) having a projecting volume adapted to cooperate with a female element (20) provided with a hollow recess which is identical in shape and complementary to the projecting volume, **characterised in that** the projecting volume (19) has a trapezoidal transverse profile extending along a longitudinal axis (Y1Y'1) and is thus in the shape of a dovetail.

2. Device (1) for injecting a liquid for medical use (2) according to claim 1, **characterised in that** the fixing element (19) has at least one planar abutment surface (21a, 21b) adapted to cooperate with a respective planar surface of an element for fixing the syringe so as to prevent pivoting between the plunger (7) and the connecting member (6), the abutment surface(s) (21a, 21b) consisting of the side walls of the volume.

3. Device (1) for injecting a liquid for medical use (2) according to claim 1 or 2, **characterised in that** it comprises a syringe holding sleeve (9) adapted to be positioned on the apparatus by sliding along a longitudinal sliding axis (ZZ') which is perpendicular to the rotation axis (XX') of the syringe, means (MP) for positioning the syringe (3) in the sleeve (9) being provided so that the longitudinal axis (Y1Y'1) or (Y2Y'2) of the male or female fixing element of the plunger (7) of the syringe coincides with the sliding axis (ZZ').

4. Device (1) for injecting a liquid for medical use (2) according to claim 3, **characterised in that** the sleeve (9) comprises, at its upper end, on the upper surface of its edge (11), a cylindrical hollow recess (16) the radius of which is substantially equal to the radius of the upper edge (17) of the syringe (3).

5. Device (1) for injecting a liquid for medical use (2) according to claim 4, **characterised in that** the means (MP) for positioning the syringe in the sleeve consist of a flat member (16a, 17a) which truncates the hollow recess (16) of the sleeve (9) in order to connect the syringe body (8) and the sleeve (9) for rotation about the rotation axis (XX'), the top edge (17) of the syringe also being truncated by a plane in the same way as the sleeve.

6. Syringe (3) intended to be used in an injection device (1) according to any of the preceding claims, **characterised in that** it comprises on the upper surface of the plunger (7) a female fixing element (20) which is formed by a hollow recess of trapezoidal transverse profile extending along a longitudinal axis (Y2Y'2) and is thus in the shape of a dovetail.

7. Syringe (3) according to claim 6, **characterised in that** the syringe body (8) comprises a top edge (17) laterally truncated by a flat member (17a) arranged parallel to the longitudinal axis (Y2Y'2) of the female fixing element (20) of the plunger (7).

8. Syringe (3) according to claim 6 or 7, **characterised in that** the plunger (7) is made of polycarbonate and the syringe body (8) is made of polypropylene.

9. Process for positioning a syringe (3) according to any one of claims 6 to 9 on the injection apparatus (4) of an injection device (1) according to any one of claims 1 to 5, **characterised in that** it comprises a main step which consists of effecting a respective rotational movement between the syringe body (8) and the plunger (7) of the syringe (3).
